# EUROPÄISCHE PATENTSCHRIFT

(11) **EP 1 638 628 B1**
(45) Veröffentlichungstag und Bekanntmachung des Hinweises auf die Patenterteilung: **25.12.2013**
(21) Anmeldenummer: 04732608.7
(22) Anmeldetag: 13.05.2004
(51) Int. Cl.: A61M 1/16, G06F 3/033, G06F 19/00

(54) **BLUTBEHANDLUNGSGERÄT**
BLOOD TREATING DEVICE
APPAREIL POUR TRAITER LE SANG

(30) Priorität: 23.05.2003 DE 10323843
(43) Veröffentlichungstag der Anmeldung: 29.03.2006
(73) Patentinhaber: Fresenius Medical Care Deutschland GmbH, 61352 Bad Homburg (DE)
(72) Erfinder: DANIEL, Pia, 78351 Bodman (DE)
(74) Vertreter: Dreyhsig, Jörg
(86) Internationale Anmeldenummer: PCT/EP2004/005115
(87) Internationale Veröffentlichungsnummer: WO 2004/103442

(56) Entgegenhaltungen:
- EP-A- 0 623 357
- WO-A-02/26288
- WO-A-96/40322

## Beschreibung

Die Erfindung betrifft ein Blutbehandlungsgerät mit einer Blutbehandlungseinrichtung, die Teil eines extrakorporalen Blutkreislaufs ist, nach dem Oberbegriff des Anspruchs 1.

Für die extrakorporale Behandlung von Blut sind unterschiedliche Geräte bekannt. Bei diesen Geräten wird Blut über eine Blutzuführleitung von einem Patienten zu einer Blutbehandlungseinrichtung geleitet und von dort über eine Blutrückführleitung wieder an den Patienten zurückgegeben. Die Blutbehandlungseinrichtung kann z.B. ein Hämodialysator oder -filter, ein Blutoxygenator, ein Blutadsorber oder eine Blutzentrifuge sein. Derartige Geräte umfassen Aktoren zur Steuerung der Blutbehandlung, die von einer Steuereinheit des Blutbehandlungsgerätes zum gezielten Ablauf der Blutbehandlung angesteuert werden.

Aufgrund vielfältiger Einsatzmöglichkeiten dieser Geräte sowie notwendiger Sicherheitsbetrachtungen sind diese Geräte relativ komplex. Für eine Bedienperson besteht dabei zunehmend die Gefahr der Unübersichtlichkeit der Anzeige- und Eingabeschnittstelle. Im Sinne der Verfügbarkeit neuer Medien werden zunehmend Touchscreens für Anzeige- und Eingabeeinheiten solcher Geräte eingesetzt. So zeigt die EP 0 904 788 A1 den Einsatz eines Touchscreens, um mit Hilfe eines graphischen Abbildes der Komponenten eines Hämodialysegerätes die Bedienbarkeit des Gerätes zu erleichtern.

US 5,609,770 offenbart eine Bedienoberfläche mit einem Touchscreen für ein Hämodialysegerät, bei dem die Parametereingabe im Mittelpunkt steht und bei dem einzelne Parameter in Gruppen eingeteilt werden. Die Eingabe von Parametern für eine Hämodialysebehandlung mit Hilfe eines Touchscreens ist auch Gegenstand der EP 0 623 357 A1. Die Eingabe und Wiedergabe bestimmter Informationen zu einer extrakorporalen Tropfenkammer bei einer Hämodialysebehandlung mit Hilfe eines Touchscreens ist Gegenstand der US 5,858,239. WO02/26288 offenbart ein Blutbehandlungsgerät nach dem Oberbegriff des Anspruchs 1.

Bei einer extrakorporalen Blutbehandlung werden mehrere, zeitlich aufeinanderfolgende Modi durchlaufen. Abgesehen von dem eigentlichen Blutbehandlungsmodus gibt es einen vorangehenden Blutbehandlungsvorbereitungsmodus und einen nachfolgenden Blutbehandlungsnachbereitungsmodus. Bei dem Blutbehandlungsvorbereitungsmodus wird der extrakorporale Blutkreislauf für den Blutbehandlungsmodus vorbereitet, indem Luft oder ein anderes Medium aus dem extrakorporalen Blutkreislauf entfernt und eine isotone Befüllflüssigkeit - im Allgemeinen Kochsalzlösung - in den extrakorporalen Blutkreislauf eingebracht wird. Am Ende einer Blutbehandlung ist das im extrakorporalen Blutkreislauf befindliche Blut dem Patienten während eines Blutbehandlungsnachbereitungsmodus zu reinfundieren.

Von den bekannten Hämodialysegeräten ausgehend hat sich die Erfindung die Aufgabe gestellt, die Bedienung eines Blutbehandlungsgerätes mit Hilfe eines Touchscreens unter spezieller Berücksichtigung der zeitlich aufeinanderfolgenden Blutbehandlungsmodi zu vereinfachen.

Nach der Lehre der Erfindung wird diese Aufgabe durch ein Blutbehandlungsgerät mit den Merkmalen des Anspruches 1 gelöst. Vorteilhafte Ausgestaltungen sind Gegenstand der Unteransprüche.

Weitere Einzelheiten und Vorteile der Erfindung werden anhand eines in den Zeichnungen dargestellten Ausführungsbeispiels näher beschrieben. Bei dieser Ausführungsform ist das Blutbehandlungsgerät ein Hämodialysegerät. Es zeigen
Figur 1 eine stark schematisierte Ansicht des Hämodialysegeräts;
Figur 2 eine erste Ansicht auf einem Touchscreen des Hämodialysegerätes mit verschiedenen Modusmitteln, wobei das Modusmittel "Blutssystem" ausgewählt ist;
Figur 3 eine zweite Ansicht auf dem Touchscreen des Hämodialysegerätes mit verschiedenen Modusmitteln, wobei das Modusmittel "Vorbereiten" ausgewählt ist;
Figur 4 eine dritte Ansicht auf dem Touchscreen des Hämodialysegerätes mit verschiedenen Modusmitteln, wobei das Modusmittel "Behandlung" ausgewählt ist;
Figur 5 eine vierte Ansicht auf dem Touchscreen des Hämodialysegerätes mit verschiedenen Modusmitteln, wobei das Modusmittel "Re-Infusion" ausgewählt ist; und
Figur 6 eine fünfte Ansicht auf dem Touchscreen des Hämodialysegerätes mit verschiedenen Modusmitteln, wobei das Modusmittel "Reinigen" ausgewählt ist.

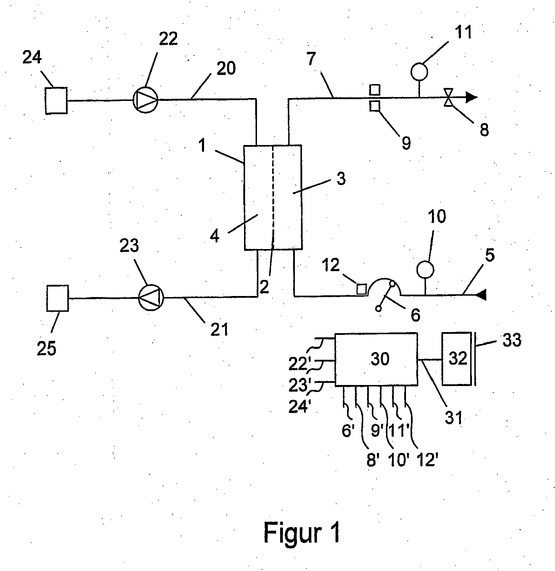

Anhand von Figur 1 wird zunächst der Aufbau eines Hämodialysegeräts kurz erläutert. Bei der Hämodialyse wird Blut in einem extrakorporalen Kreislauf über eine Blutzuführleitung 5 einem als Hämodialysator 1 ausgeführten Blutreinigungselement zugeführt. Im Hämodialysator 1 trennt eine meist in Form vieler Hohlfasern ausgeführte semipermeable Membran 2 eine erste Kammer 3, die Teil des extrakorporalen Blutkreislaufs ist, von einer zweiten Kammer 4, die Teil eines Dialysierflüssigkeitkreislaufes ist. Durch die semipermable Membran 2 treten aus dem Blut zu entfernende Stoffe in die Dialysierflüssigkeit über, die durch diese abgeführt werden. Gleichzeitig kann über einen Druckgradienten eine überschüssige Flüssigkeitsmenge aus dem Blut ultrafiltriert und über die abfließende Dialysierflüssigkeit entfernt werden.

In der Blutzuführleitung 5 wird Blut durch eine als Rollenpumpe ausgestaltete Blutpumpe 6 gefördert. Das Blut verlässt die erste Kammer 3 des Hämodialysators 1 über die Blutrückführleitung 7, um wieder zum Patienten zurückgeführt zu werden. An der Blutrückführleitung 8 ist eine venöse Absperrklemme 8 vorgesehen, mit der die Rückführung von Blut insbesondere in Notfällen unterbrochen werden kann. Derartige Notfälle können z.B. auftreten, wenn durch einen Luftdetektor 9 Luft in der Blutrückführleitung 7 detektiert wird. Der Luftdetektor 9 umfasst auch Mittel, um die Präsenz von Blut in der Blutrückführleitung 7 zu erkennen.

An der Blutzuführleitung 5 ist ein arterieller Drucksensor 10 und an der Blutrückführleitung 7 ein venöser Drucksensor 11 vorgesehen.

Die zweite Kammer 4 des Hämodiälysators wird von Dialysierflüssigkeit durchströmt, die über eine Dialysierflüssigkeitszuführleitung 20 von einer Dialysierflüssigkeitsquelle 24 zugeführt und über eine Dialysierflüssigkeitsabführleitung 21 zu einem Abfluss 25 abgeführt wird. Die Dialysierflüssigkeit wird durch Förder- und Bilanziereinrichtungen 22 und 23 umgewälzt, wobei ein eventuell zu entfernendes Ultrafiltrat in der Menge genau erfasst werden kann. Dem Fachmann stehen zur Realiserung der Förder- und Bilanziereinrichtungen 22 und 23 verschiedenste Ausgestaltungen zur Verfügung, so dass an dieser Stelle von näheren Ausführungen abgesehen wird. Gleiches gilt auch für die Bereitstellung von Dialysierflüssigkeit durch die Dialysierflüssigkeitsquelle 24. Beispielhaft wird an dieser Stelle auf ein Bilanzkammersystem hingewiesen, wie es in der US 4,267,040 beschrieben ist.

Auch zum Einsatz von Aktoren und Sensoren in einem Hämodialysegerät im Allgemeinen stehen dem Fachmann zahlreiche Möglichkeiten zur Verfügung, ohne dass hier im Detail darauf eingegangen werden muss. Die Darstellung in Figur 1 ist auf einige wenige dieser Elemente beschränkt, die für die Erläuterung der Erfindung ausreichend sind.

Das Hämodialysegerät wird durch eine Steuereinheit 30 gesteuert und überwacht. Hierzu ist die Steuereinheit 30 mit den einzelnen Aktoren und Sensoren des Gerätes mit Signalleitungen verbunden. Für die in Figur 1 dargestellten Aktoren und Sensoren ist dies durch Bezugsziffern angedeutet, die neben der Bezugsziffer des betreffenden Aktors oder Sensors ein Apostroph aufweisen und die der Übersichtlichkeit halber nur an der Steuereinheit 30 eingezeichnet sind.

Die Steuereinheit 30 ist mit einer Anzeige- und Eingabeeinheit 32 über eine Datenleitung 31 verbunden. Die Anzeige- und Eingabeeinheit 32 umfasst einen Touchscreen 33. Auf dem Touchscreen werden von der Steuereinheit 30 gemeldete Informationen angezeigt, gleichzeitig werden über den Touchscreen von einer Bedienperson eingegebene Daten an die Steuereinheit 30 weitergeleitet.

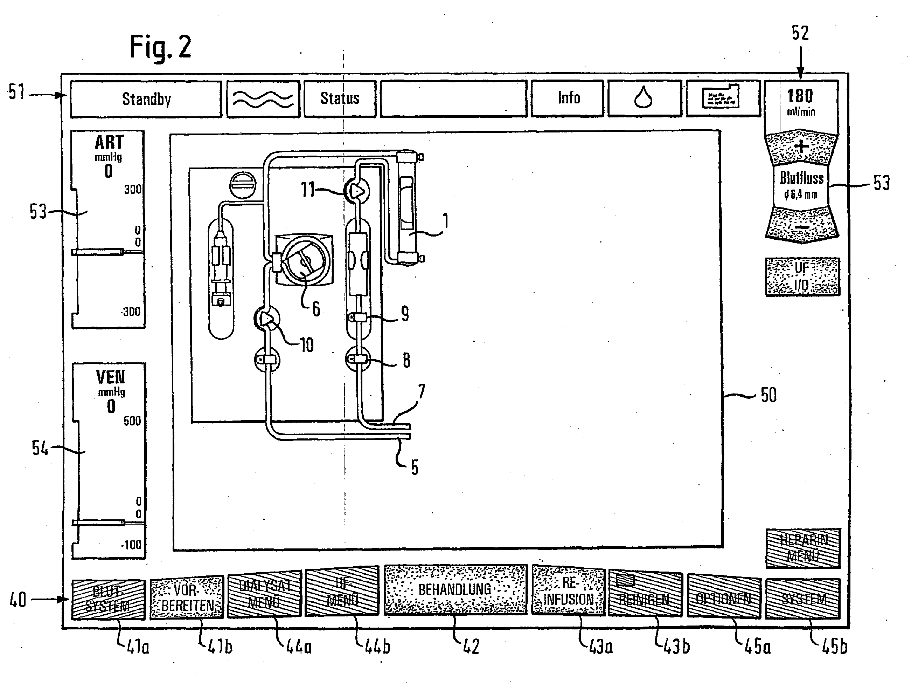

Figur 2 zeigt eines erste Ansicht des Touchscreens 33 des Hämodialysegerätes. Am unteren Rand des Touchscreens sind verschiedene Modusmittel 40 in einer nebeneinanderliegenden Reihe dargestellt. Die Modusmittel 40 umfassen verschiedene Arten von Modusmitteln. Zunächst gibt es Blutbehandlungsvorbereitungsmittel 41 a und 41 b, Blutbehandlungsmittel 42 und Blutbehandlungsnachbereitungsmittel 43a und 43b, d.h. diese Modusmittel betreffen zeitliche Modi vor einer Blutbehandlung - hier einer Hämodialysebehandlung - , die eigentliche Blutbehandlung und nach einer Blutbehandlung. In jedem dieser Modi durchläuft das Hämodialysegerät spezifische Verfahrensschritte, wobei die Modi in einer bestimmten zeitlichen Reihenfolge ablaufen.

Des Weiteren sind Ergänzungsmodusmittel 44a, 44b und 45a und 45b vorgesehen, die die Eingabe ergänzender Informationen zu mehreren Zeitpunkten ermöglichen. Die Bedeutung der einzelnen Modi wird im Fortgang der Beschreibung näher beschrieben.

Auf dem Touchscreen 33 ist über den Modusmitteln 40 ein Bereich 50 angeordnet, auf dem je nach Betriebsmodus unterschiedliche Ansichten zu sehen sind. In den Randbereichen 51, 52 sind weitere Eingabe- und/oder Ausgabemittel (z.B. Mittel 53 für die Blutpumpe 6) vorgesehen, die bestimmte Dateneingaben und die Anzeige erwünschter Informationen ermöglichen. Diese Randbereiche können unabhängig vom Betriebsmodus einen gleichartigen Aufbau haben oder davon abhängig sein. Da dieser Teil des Touchscreens für die Erläuterung der Erfindung von untergeordneter Bedeutung ist, wird darauf an dieser Stelle nicht näher eingegangen.

Am Beginn einer Hämodialysebehandlung schaltet das Hämodialysegerät in den Modus "Blutsystem" (Figur 2). Wenn das Gerät durch einen geeigneten Sensor, z.B. einen mechanischer Berührungssensor 12 an der Blutpumpe 6, keinen eingelegten Blutschlauch erkennt, wird dieser Modus von der Steuereinheit 30 automatisch angezeigt. Anderfalls wird er übersprungen, und die Steuereinheit 30 veranlasst die Auswahl des zeitlich nachfolgenden Vorbereiten-Modus.

Im Blutsystem-Modus wird auf der Anzeigefläche 50 ein graphisches Abbild eines an dem Hämodialysegerät montierten Blutschlauchstems dargestellt, um dem Anwender das Einlegen eines Blutschlauchsystems zu erleichtern. Dabei sind entsprechend der Figur 1 der Hämodialysator 1, die Blutzuführleitung 5, die Blutpumpe 6, der arterielle Drucksensor 10, die Blutrückführleitung 7, die venöse Klemme 8, der Luft- und Blutdetektor 9 und der venöse Drucksensor 11 zu erkennen.

Die Anzeige- und Eingabeeinheit 32 kann die Modusmittel 40 auf dem Touchscreen 33 in drei Symbolarten darstellen. Zunächst werden in einer ersten Symbolart diejenigen Modusmittel dargestellt, die im momentanen Modus manuell über den Touchscreen 33 auswählbar sind. Für die in Figur 2 gezeigte Ansicht trifft dies auf die Modusmittel 44a, 44b, 43b und 45 zu. In einer zweiten Symbolart wird das Modusmittel angezeigt, das den momentanen ausgewählten Modus anzeigt (Blutsystem-Modusmittel 41 a in Figur 2). Eine dritte Symbolart wird für die verbleibenden Modusmittel verwendet, die im momentanen Modus deaktiviert sind. In Figur 2 sind dies die Modusmittel 41 b, 42 und 43a.

An der linken Seite der Ansicht des Touchscreens werden in Form von Balkenanzeigen 52 und 53 die Messwerte des arteriellen und venösen Drucksensors 10 und 11 angezeigt.

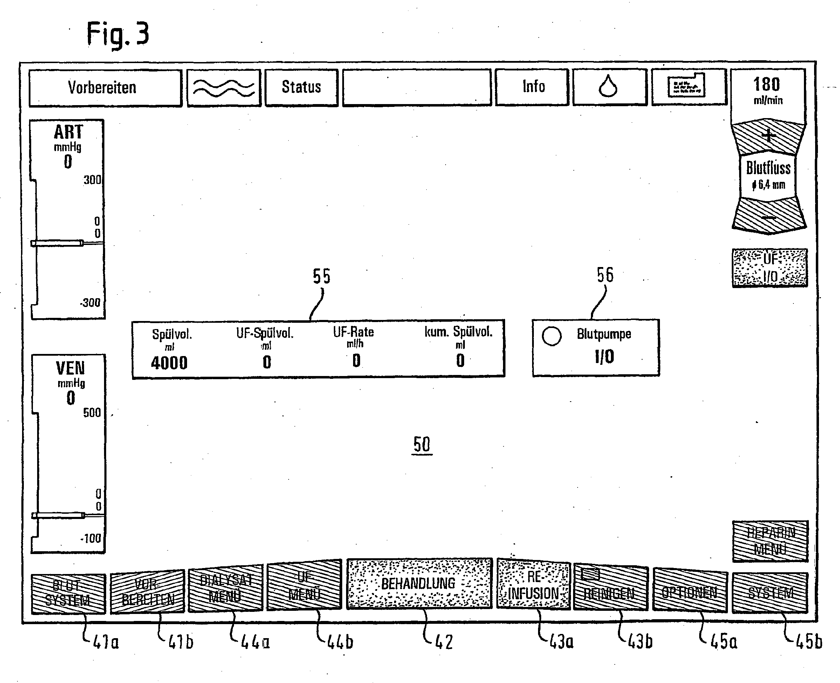

Nachdem die Steuereinheit 30 die Anwesenheit eines ordnungsgemäß eingelegten Schlauchsystems mit Hilfe des Sensors 12 detektiert hat, veranlasst sie erfindungsgemäß die Beendigung des Blutsystem-Modus und den Beginn des zeitlich anschließenden Vorbereiten-Modus. Die Anzeige- und Eingabeeinheit 32 wird dabei angewiesen, die Modusmittel 40 entsprechend darzustellen. Nun wird automatisch das Modusmittel 41 b ausgewählt und die Ansicht gemäß Figur 3 geändert.

Die Ansicht im Bereich 50 des Touchscreens 33 zeigt nun in der Datenleiste 55 eine Ansicht von Parametern, wie sie für den Verlauf der Spülung des Blutschlauchsystems repräsentativ sind. Bei der Spülung wird z.B. ein Beutel mit physiologischer Kochsalzlösung an die Blutzuführleitung 5 angeschlossen. Die Blutrückführleitung 7 führt zu einem Abfluss. Zur ausreichenden Spülung stehen mindestens 4 Liter Kochsalzlösung bereit. Durch Betätigung des Blutpumpenaktivierungsmittels 56 wird die Blutpumpe mit einem vorher eingestellten Förderfluss eingeschaltet. Die Daten in der Datenleiste 55 zeigen danach die jeweils aktuellen Werte an. Ist eine ausreichende Spülflüssigkeitsmenge durch den extrakorporalen Blutkreislauf gefördert worden, wird die Blutpumpe 6 durch die Steuereinheit 30 automatisch bei Erreichen des vorgegebenen Spülzielvolumens gestoppt. Es ist auch möglich, die Spülung manuell durch die Blutpumpenaktivierungsmittel 56 zu beenden, sollte ein kleineres Spülvolumen als ausreichend erachtet werden.

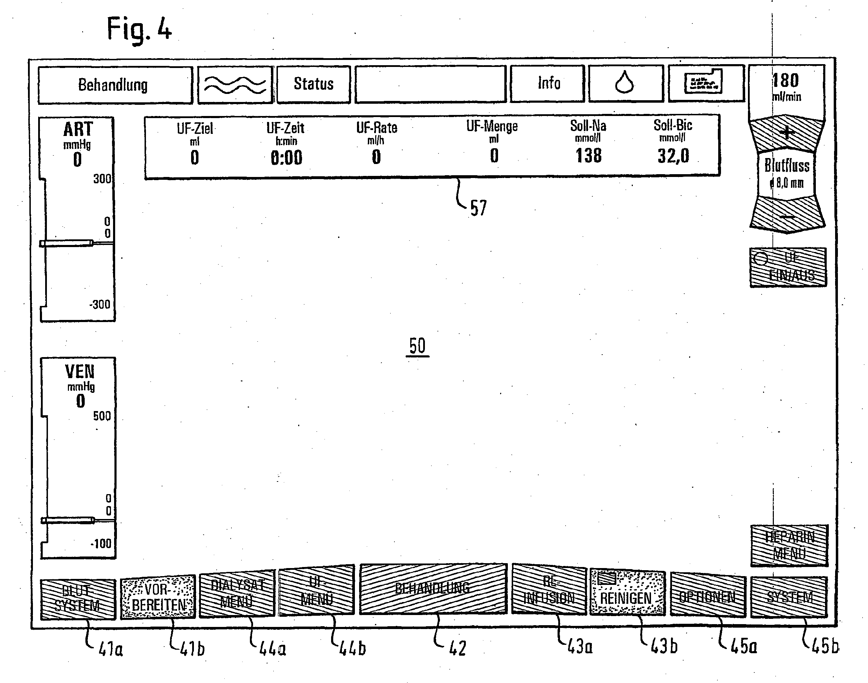

Die Bedienperson schließt nun die Blutzuführleitung 5 und Blutrückführleitung 7 an ein Gefäß des Patienten an. Die Blutpumpe 6 muss danach wieder mit Hilfe der Blutpumpenaktivierungsmittel 56 in Betrieb gesetzt werden. Sobald Blut im Blutdetektor 9 erkannt wird, kann die Blutbehandlung beginnen. Der Blutdetektor 9 weist zu diesem Zweck einen optischen Detektor auf, der die Färbung eines Mediums in der Blutrückführleitung 7 durch geeignete Wahl der Wellenlänge im Durchlichtverfahren untersucht. Das entsprechende Signal wird von der Steuereinheit 30 emfangen die dadurch erfindungsgemäß den nächsten zeitlich sich anschließenden Betriebsmodus auswählt und dies der Anzeige- und Eingabeeinheit 32 zur entsprechenden Anzeige mitteilt. Dann zeigt sich auf dem Touchscreen 33 die in Figur 4 gezeigte Ansicht.

Der nun automatisch eingeleitete zeitliche Modus ist der Behandlung-Modus, d.h. die eigentliche Hämodialysebehandlung beginnt. Im Behandlung-Modus wird auf der Anzeigefläche 50 die Datenleiste 57 angezeigt. Datenleiste 57 gibt Behandlungsfortschrittparameter wie die vergangene Behandlungszeit und die bereits entzogene Ultrafiltrationsmenge wieder. Außerdem werden Eckwerte der Dialysierflüssigkeitszusammensetzung wie die Natrium- und die Bicarbonatkonzentration angezeigt.

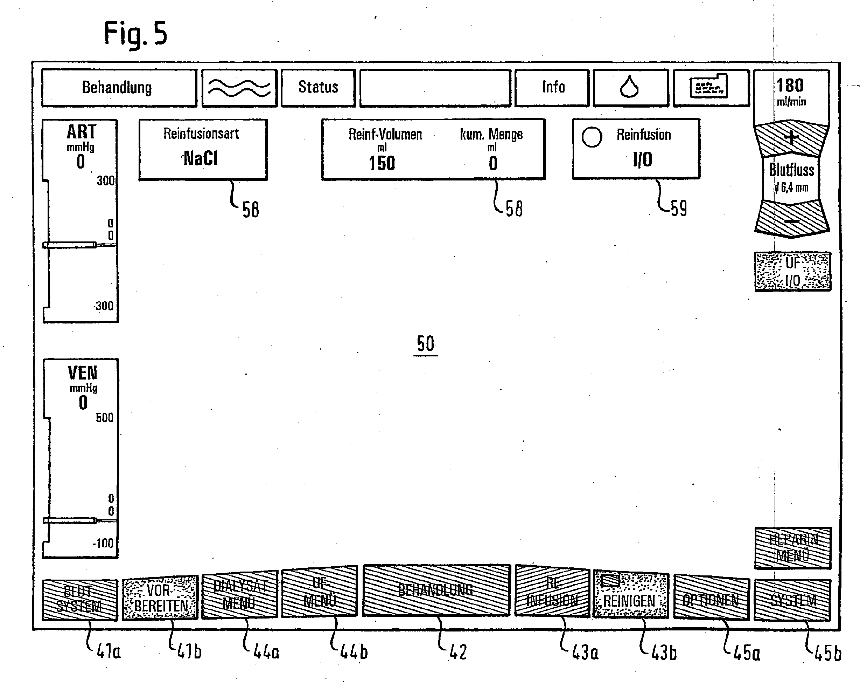

Wird ein vorgegebenes Behandlungsziel erreicht, z.B. eine insgesamt zu entziehende Ultrafiltrationsmenge, so wird die Hämodialysebehandlung von der Steuereinheit 30 durch Anhalten der Blutpumpe 6 gestoppt. Erfindungsgemäß wird von der Steuereinheit 30 außerdem der sich zeitlich anschließende Modus der Reinfusion eingeleitet. Dazu wird die Anzeige- und Eingabeeinheit 32 von der Steuereinheit 30 angewiesen, die Ansicht gemäß Figur 5 zu wechseln.

Das Bedienpersonal trennt nun die Blutzuführleitung 5 vom Patienten und schließt den Beutel mit isotonischer Kochsalzlösung erneut an diese Leitung an. In der Datenleiste 58 werden analog zur Datenleiste 55 Angaben über die zu erfolgende ReInfusion angezeigt. Mit Hilfe des Blutpumpenaktivierungsmittels 59 kann die ReInfusion gestartet werden. Die Steuereinheit 30 steuert die Blutpumpe 6 dergestalt, dass ein vorgegebenes Blutschlauchvolumen von in diesem Beispiel 150 ml gefördert wird, um dem Patienten das im extrakorporalen Kreislauf befindliche Blut über die Blutrückführleitung 7 zurückzugeben. Nach Förderung der vorgegebenen Flüssigkeitsmenge leitet die Steuereinheit 30 erfindungsgemäß den nächsten sich zeitlich anschließenden Modus - den Reinigen-Modus - ein.

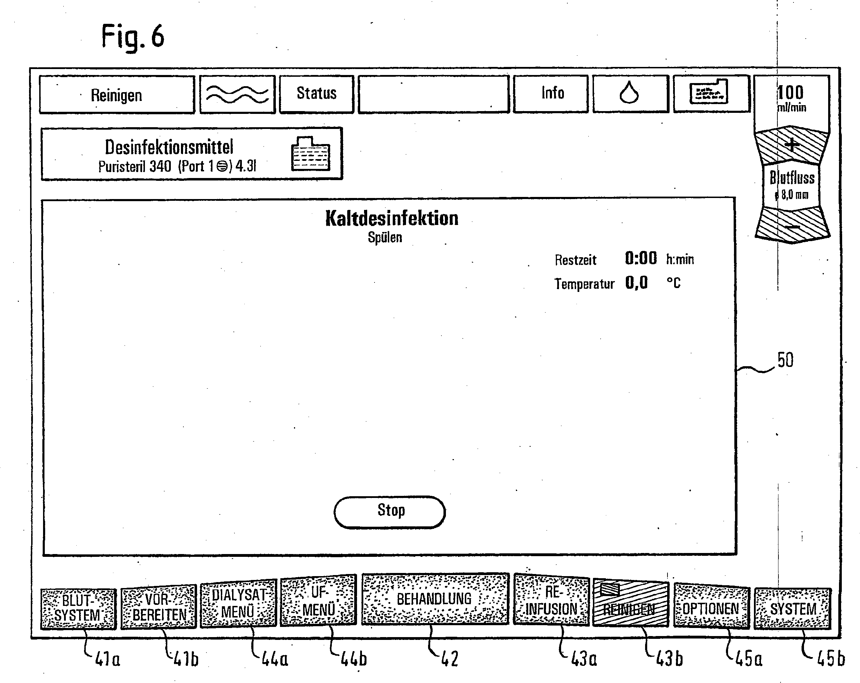

Im Reinigen Modus zeigt die Anzeige- und Eingabeeinheit 32 die Ansicht an, die in Figur 6 zu sehen ist. Der Patient wird nun vom Bedienpersonal vollständig vom Hämodialysegerät getrennt, bevor Spül- und Desinfektionsschritte eingeleitet werden. Während der Reinigung werden Betriebsparameter auf der Anzeigefläche 50 dargestellt. Das Hämodialysegerät ist damit am Ende der einzelnen zeitlichen Betriebsmodi für eine Hämodialysebehandlung angelangt.

Die erfindungsgemäße Ausgestaltung des Hämodialysegerätes vereinfacht durch die beschriebene automatische Auswahl der Modusmittel bei Einleitung sich zeitlich anschließender Betriebsmodi die Bedienerführung und hilft dadurch, Fehlbedienungen zu vermeiden. Zusätzlich wird durch eine der zeitlichen Abfolge entsprechenden Anordnung der Modusmittel die Übersichtlichkeit der Touchscreenelemente verbessert. Dies gilt auch für die permanente Sichtbarkeit der einzelnen Modusmittel in allen Ansichten unabhängig vom Betriebsmodus, was eine besonders vorteilhafte Ausführungsform darstellt. Durch das automatische Auswählen sich anschließender Betriebsmodi wird das Bedienpersonal zudem entlastet, da weniger Eingaben erforderlich ist. Dies gilt insbesondere in den Fällen, in denen automatisch neben der Änderung der Darstellung auf dem Touchscreen bei Beginn eines sich anschließenden zeitlichen Modus auch Aktoren des Blutbehandlungsgeräts selbst in Gang gesetzt werden. In dem vorgestellten Beispiel war dies beim Übergang vom Vorbereiten-Modus zum Behandlung-Modus der Fall.

Zur Erhöhung der Übersichtlichkeit kann bei der Ansteuerung des Touchscreens vorgesehen sein, die für die einzelnen Modusmittel zu verwendenden Symbole in ihrem Erscheinungsbild zu variieren. Die Modusmittel 40 in den Figuren 2 bis 6 weisen demnach eine variierende Höhe auf, die beim Blutbehandlungsmittel 42 den größten Wert hat. Zu den Blutbereitungsvorbereitungsmitteln 41a und 41 b sowie den Blutbehandlungsnachbereitungsmitteln 42a und 42b hin fällt die Höhe ab, um diesen drei zeitlichen Bereichen ein zusätzliche Anschaulichkeit zu verleihen, indem die Blutbehandlungsmittel 42 hervorgehoben werden.

Die Modusmittel 40 können weitere, bisher nicht erwähnte Ergänzungsmodusmittel 44a, 44b, 45a und 45b umfassen, In dem in den Figuren 2 bis 6 gezeigten Beispiel können mit Hilfe des Dialysat-Modusmittels 44a und des Ultrafiltrat-Modusmittel 44b von Behandlungsbasiswerten abweichende Behandlungswerte eingegeben werden. Eine manuelle Auswahl dieser Modusmittel bietet sich insbesondere während der Vorbereitung einer Hämodialysebehandlung an. Aus diesem Grund sind diese Modusmittel auf der Blutbehandlungsvorbeitungsseite der Modusmittel 40 angeordnet. Es ist allerdings auch möglich, die entsprechenden Behandlungsparameter wie z.B. Dialysierflüssigkeitfluss, Ultrafiltrafiltrationsmenge, Behandlungszeit etc. auch noch während einer Hämodialysebehandlung vor dem Behandlungsende manuell durch Auswahl dieser Modusmittel zu ändern.

Das Optionen-Modusmittel 45a ist für Erweiterungsfunktionen des Hämodialysegerätes reserviert, die ebenfalls den Verlauf einer Hämodialysebehandlung beeinflussen oder zumindest weitere Messdaten zu deren Überwachung zur Verfügung stellen. Die System-Modusmittel 45b betreffen Geräteeinstellungen wie die Lautstärke eines Lautsprechers oder die Helligkeit der Anzeige, die jederzeit geändert werden können und die in keinem direkten Zusammenhang zur Blutbehandlung stehen.

## Patentansprüche

1. Blutbehandlungsgerät mit einer Blutbehandlungseinrichtung (1), die Teil eines extrakorporalen Blutkreislaufs ist,
mit Aktoren (6, 8, 22, 23, 24) im extrakorporalen Blutkreislauf und/oder in weiteren Flüssigkeitskreisläufen,
mit einer Steuereinheit (30) zum Steuern der Aktoren (6, 8, 22, 23, 24),
mit einer mit der Steuereinheit (30) verbundenen, einen Touchscreen (33) umfassenden Anzeige- und Eingabeeinheit (32),
wobei die Anzeige- und Eingabeeinheit (32) verschiedene Modusmittel (40) umfasst, um auf dem Touchscreen (33) verschiedene zeitliche Modi einer Blutbehandlung darstellen zu können, wobei die Modusmittel (40) über den Touchscreen (33) von einer Bedienperson auswählbar sind,
wobei die Steuereinheit (30) geeignet ist, den jeweils laufenden zeitlichen Modus zu identifizieren und die Anzeige- und Eingabeeinheit (32) anzuweisen, das entsprechende Modusmittel von den anderen Modusmitteln ausgewählt darzustellen, indem die anderen Modusmittel in einer ersten Symbolart und das ausgewählte Modusmittel in einer zweiten Symbolart dargestellt werden,
**dadurch gekennzeichnet,**
**dass** die Steuereinheit (30) ferner geeignet ist, das Ende zumindest eines zeitlichen Modus festzustellen, um automatisch den Beginn des anschließenden zeitlichen Modus einzuleiten sowie dies der Anzeige- und Eingabeeinheit (32) zum Wechsel der Darstellung des ausgewählten Modusmittels mitzuteilen,
**dass** die Modusmittel (40) in ihrer zeitlichen Abfolge zueinander angeordnet sind und.
**dass** die Modusmittel (40) mindestens ein Blutbehandlungsvorbereitungsmittel (41 a, 41b), ein Blutbehandlungsmittel (42) und ein Blutbehandlungsnachbereitungsmittel (43a, 43b) umfassen.

2. Blutbehandlungsgerät nach Anspruch 1, **dadurch gekennzeichnet, dass** das Blutbehandlungsmittel (42) auf dem Touchscreen (33) eine größere Fläche aufweist als die anderen Modusmittel (41a, 41b, 43a, 43b).

3. Blutbehandlungsgerät nach Anspruch 1, **dadurch gekennzeichnet, dass** die Modusmittel (40) in Form einer Zeile an einem Rand des Touchscreens (33) dargestellt werden und die verbleibende Fläche des Touchscreens zur Darstellung weiterer Ausgabe- und/oder Eingabemittel geeignet ist.

4. Blutbehandlungsgerät nach einem der vorhergehenden Ansprüche, **dadurch gekennzeichnet, dass** der Touchscreen (33) eine Anzeigefläche (50) aufweist, auf der die Anzeige- und Eingabeeinheit (32) in Anhängigkeit vom zeitlichen Modus verschiedene Ausgabe- und/oder Eingabemittel (55, 56, 57, 58, 59) darstellt.

5. Blutbehandlungsgerät nach Anspruch 1, **dadurch gekennzeichnet, dass** das Blutbehandlungsgerät ein Hämodialysegerät ist.

6. Blutbehandlunggerät nach Anspruch 5, **dadurch gekennzeichnet, dass** die mindestens einen Blutbehandlungsvorbereitungsmittel je Modusmittel für einen Blutsystem-Modus (41a) und einen Vorbereiten-Modus (41b) umfassen.

7. Blutbehandlungsgerät nach Anspruch 6, **dadurch gekennzeichnet, dass** die mindestens einen Blutnachbereitungsmittel je Modusmittel für einen ReInfusion-Modus (43a) und einen Reinigen-Modus (43b) umfassen.

8. Blutbehandlungsgerät nach einem der vorhergehenden Ansprüche, **dadurch gekennzeichnet, dass** die Steuereinheit (30) geeignet ist, die Anzeige- und Eingabeeinheit (32) anzuweisen, je nach dem laufenden zeitlichen Modus einzelne Modusmittel in einer dritten Symbolart darzustellen und deren Eingabefunktion zu deaktivieren.

9. Blutbehandlungsgerät nach einem der vorhergehenden Ansprüche, **dadurch gekennzeichnet, dass** die Anzeige- und Eingabeeinheit (32) geeignet ist, die Modusmittel (40) in allen zeitlichen Modi an der gleichen Stelle des Touchscreens (33) anzuzeigen.

10. Blutbehandlungsgerät nach einem der vorhergehenden Ansprüche, **dadurch gekennzeichnet, dass** das Blutbehandlungsgerät Sensoren (9, 10, 11, 12) umfasst, wobei die Steuereinheit (30) geeignet ist, die Messwerte der Sensoren auszuwerten, um das Ende eines zeitlichen Modus festzustellen.

11. Blutbehandlungsgerät nach Anspruch 10, **dadurch gekennzeichnet, dass** der Sensor ein Blutdetektor und/oder Luftdetektor (9) im extrakorporalen Kreislauf ist.

12. Blutbehandlungsgerät nach Anspruch 10, **dadurch gekennzeichnet, dass** der Sensor ein Detektor (12) ist, der die Präsenz von ordungsgemäß montierten Komponenten des extrakorporalen Kreislaufs feststellt.

13. Blutbehandlungsgerät nach einem der vorhergehenden Ansprüche, **dadurch gekennzeichnet, dass** die Steuereinheit geeignet ist, die durch eine angesteuerte Pumpe (6, 22, 23) in einer bestimmten Zeit geförderte Flüssigkeitsmenge zu bestimmen, um diesen Wert zur Bestimmung des Endes eines zeitlichen Modus heranzuziehen.

## Claims

1. A blood treatment machine having a blood treatment device (1) which is part of an extracorporeal blood circulation,
having actuators (6, 8, 22, 23, 24) in the extracorporeal blood circulation and/or in other blood circulations,
having a control unit (30) for controlling the actuators (6, 8, 22, 23, 24),
having a display and input unit (32) comprising a touchscreen (33) and connected to the control unit (30),
wherein the display and input unit (32) comprises various mode means (40) for being able to display various time modes of a blood treatment on the touchscreen (33), the mode means (40) being selectable by an operator via the touchscreen (33),
wherein the control unit (30) is suitable for identifying the time mode running in each case and for instructing the display and input unit (32) to display the corresponding mode means as selected from the other mode means by displaying the other mode means in a first type of symbol and displaying the selected mode means in a second type of symbol,
**characterized in that**
the control unit (30) is also suitable for detecting the end of at least one time mode to automatically initiate the start of the subsequent time mode and to send this information to the display and input unit (32) for changing the display of the selected mode means,
the mode means (40) are arranged in their chronological sequence in relation to one another, and
the mode means (40) comprise at least one blood treatment preparatory means (41a, 41b), a blood treatment means (42) and a blood treatment post-preparation means (43a, 43b).

2. The blood treatment machine according to Claim 1, **characterized in that** the blood treatment means (42) have a larger area on the touchscreen (33) than the other mode means (41a, 41b, 43a, 43b).

3. The blood treatment machine according to Claim 1, **characterized in that** the mode means (40) are represented in the form of a line on one edge of the touchscreen (33), and the remaining area of the touchscreen is suitable for displaying other input and/or output means.

4. The blood treatment machine according to any one of the preceding claims, **characterized in that** the touchscreen (33) has a display area (50) on which the display and input unit (32) displays various input and/or output means (55, 56, 57, 58, 59) as a function of the time mode.

5. The blood treatment machine according to Claim 1, **characterized in that** the blood treatment machine is a haemodialysis machine.

6. The blood treatment machine according to Claim 5, **characterized in that** the at least one blood treatment preparatory means each comprise mode means for both a blood system mode (41a) and a preparatory mode (41b).

7. The blood treatment machine according to Claim 6, **characterized in that** the at least one blood treatment post-preparation means each comprise mode means for both a reinfusion mode (43a) and a cleaning mode (43b).

8. The blood treatment machine according to any one of the preceding claims, **characterized in that** the control unit (30) is suitable for instructing the display and input unit (32) to display individual mode means in a third type of symbol, depending on the ongoing time mode and to deactivate their input function.

9. The blood treatment machine according to any one of the preceding claims, **characterized in that** the display and input unit (32) is suitable for displaying the mode means (40) in all time modes at the same location on the touchscreen (33).

10. The blood treatment machine according to any one of the preceding claims, **characterized in that** the blood treatment device includes sensors (9, 10, 11, 12), the control unit (30) being suitable for evaluating the measured values of the sensors to determine the end of a time mode.

11. The blood treatment machine according to Claim 10, **characterized in that** the sensor is a blood detector and/or air detector (9) in the extracorporeal circulation.

12. The blood treatment machine according to Claim 10, **characterized in that** the sensor is a detector (12), which determines the presence of properly installed components of the extracorporeal circulation.

13. The blood treatment machine according to any one of the preceding claims, **characterized in that** the control unit is suitable for determining the quantity of fluid conveyed by a triggered pump (6, 22, 23) in certain period of time to use this value for determining the end of a time mode.

## Revendications

1. Appareil de traitement du sang comprenant un dispositif de traitement du sang (1) qui fait partie d'un circuit sanguin extracorporel,
comprenant des actionneurs (6, 8, 22, 23, 24) dans le circuit sanguin extracorporel et/ou dans d'autres circuits sanguins extracorporels,
comprenant une unité de commande (30) pour actionner les actionneurs (6, 8, 22, 23, 24),
comprenant une unité d'affichage et de saisie (32) comprenant un écran tactile (33), reliée à l'unité de commande (30),
sachant que l'unité d'affichage et de saisie (32) comprend différents moyens de mode (40) pour pouvoir afficher sur l'écran tactile (33) différents modes temporels d'un traitement du sang, sachant que les moyens de mode (40) peuvent être sélectionnés par un opérateur via l'écran tactile (33),
sachant que l'unité de commande (30) est appropriée pour identifier le mode temporel respectif en cours et ordonner à l'unité d'affichage et de saisie (32) d'afficher le moyen de mode correspondant sélectionné parmi les autres moyens de mode, en ce que les autres moyens de mode sont affichés dans un premier type de symbole et le moyen de mode sélectionné est affiché dans un deuxième type de symbole,
**caractérisé en ce que**,
l'unité de commande (30) est en outre appropriée pour détecter la fin au moins d'un mode temporel pour lancer automatiquement le début du mode temporel suivant ainsi que pour le communiquer à l'unité d'affichage et de saisie (32) pour changer l'affichage du moyen de mode sélectionné,
que les moyens de mode (40) sont agencés entre eux dans leur suite temporelle et
que les moyens de mode (40) comprennent au moins un moyen de préparation du traitement du sang (41a, 41b), un moyen de traitement du sang (42) et un moyen de poursuite du traitement (43a, 43b).

2. Appareil de traitement du sang selon la revendication 1, **caractérisé en ce que** le moyen de traitement du sang (42) présente une plus grande surface que les autres moyens de mode (41a, 41b, 43a, 43b) sur l'écran tactile (33).

3. Appareil de traitement du sang selon la revendication 1, **caractérisé en ce que** les moyens de mode (40) sont affichés sous forme d'une ligne sur un bord de l'écran tactile (33) et la surface restante de l'écran tactile est appropriée pour afficher des moyens de sortie et/ou d'entrée supplémentaires.

4. Appareil de traitement du sang selon l'une des revendications précédentes, **caractérisé en ce que** l'écran tactile (33) présente une surface d'affichage (50) sur laquelle l'unité d'affichage et de saisie (32) affiche différents moyens de sortie et/ou d'entrée (55, 56, 57, 58, 59) en fonction du mode temporel.

5. Appareil de traitement du sang selon la revendication 1, **caractérisé en ce que** l'appareil de traitement du sang est un appareil d'hémodialyse.

6. Appareil de traitement du sang selon la revendication 5, **caractérisé en ce que** les au moins un moyen de préparation du traitement du sang comprennent des moyens de mode respectifs pour un mode de système sanguin (41a) et un mode de préparation (41b).

7. Appareil de traitement du sang selon la revendication 6, **caractérisé en ce que** les au moins un moyen de poursuite du traitement du sang comprennent des moyens de mode respectifs pour un mode de reperfusion (43a) et un mode de nettoyage (43b).

8. Appareil de traitement du sang selon l'une des revendications précédentes, **caractérisé en ce que** l'unité de commande (30) est appropriée pour ordonner à l'unité d'affichage et de saisie (32) d'afficher différents moyens de mode dans un troisième type de symbole en fonction du mode temporel en cours et de désactiver leurs fonctions de saisie.

9. Appareil de traitement du sang selon l'une des revendications précédentes, **caractérisé en ce que** l'unité d'affichage et de saisie (32) est appropriée pour afficher les moyens de mode (40) au même endroit sur l'écran tactile (33) dans tous les modes temporels.

10. Appareil de traitement du sang selon l'une des revendications précédentes, **caractérisé en ce que** l'appareil de traitement du sang comprend des capteurs (9, 10, 11, 12), sachant que l'unité de commande (30) est appropriée pour évaluer les valeurs de mesure des capteurs afin de détecter la fin d'un mode temporel.

11. Appareil de traitement du sang selon la revendication 10, **caractérisé en ce que** le capteur est un détecteur de sang et/ou un détecteur d'air (9) dans le circuit extracorporel.

12. Appareil de traitement du sang selon la revendication 10, **caractérisé en ce que** le capteur est un détecteur (12) qui détecte la présence de composants du circuit extracorporel montés conformément.

13. Appareil de traitement du sang selon l'une des revendications précédentes, **caractérisé en ce que** l'unité de commande est appropriée pour déterminer la quantité de liquide transportée en un temps défini par une pompe pilotée (6, 22, 23) pour se servir de cette valeur afin de déterminer la fin d'un mode temporel.
